# EUROPÄISCHE PATENTSCHRIFT

(11) **EP 2 804 504 B1**
(45) Veröffentlichungstag und Bekanntmachung des Hinweises auf die Patenterteilung: **07.06.2017**
(21) Anmeldenummer: 12788537.4
(22) Anmeldetag: 21.11.2012
(51) Int. Cl.: A45D 34/04, B05B 1/00, B65D 83/20

(54) **MEDIENSPENDER**
MEDIA DISPENSER
DISTRIBUTEUR DE FLUIDES

(30) Priorität: 16.01.2012 DE 102012200545
(43) Veröffentlichungstag der Anmeldung: 26.11.2014
(73) Patentinhaber: Aptar Radolfzell GmbH, 78315 Radolfzell (DE)
(72) Erfinder: GREINER-PERTH, Jürgen, 78244 Gottmadingen (DE); KÖNIG, Peter, 78239 Rielasingen-Worblingen (DE); SCHWARZ, Nicole, 78315 Radolfzell (DE)
(74) Vertreter: Patentanwaltskanzlei Cartagena
(86) Internationale Anmeldenummer: PCT/EP2012/073210
(87) Internationale Veröffentlichungsnummer: WO 2013/107541

(56) Entgegenhaltungen:
- WO-A1-01/25116
- WO-A1-2006/013353
- WO-A1-2006/129039
- WO-A2-01/44076
- FR-A1- 2 900 645
- GB-A- 1 221 191
- US-A- 4 187 963

## Beschreibung

### Anwendungsgebiet und Stand der Technik

Die Erfindung betrifft einen Spender zum Austrag insbesondere Flüssiger Medien nach dem Oberbegriff von Anspruch 1.

Gattungsgemäße Spender sind aus dem Stand der Technik allgemein bekannt. Bei gattungsgemäßen Spendern sind die zwei genannten Teileinheiten, die Basiseinheit und der Austragkopf, vorgesehen, die im Zuge der Montage des Spenders miteinander verbunden werden. Zu diesem Zweck wird der Austragkopf derart mit der Basiseinheit gekoppelt, dass nach erfolgter Kopplung mittels einer Bewegung des Austragkopfes der Auslassstutzen der Basiseinheit bewegt werden kann.

Obwohl viele bekannte Spender die genannten Merkmale eines gattungsgemäßen Spenders erfüllen, betrifft die Erfindung insbesondere solche gattungsgemäßen Spender, bei denen der Medienspeicher der Basiseinheit zur Lagerung von permanent druckbeaufschlagten Medium vorgesehen ist und bei denen die Bewegung des Auslassstutzens gegenüber den Medienspeicher das Öffnen eines Auslassventils verursacht, so dass das Medium durch den Auslassstutzen hindurch aus dem Mediumspeicher herausströmen kann. Solche gattungsgemäßen Spender sind beispielsweise in Form von Deodorant-Spendern allgemein bekannt.

Neben Spendern aus dem Bereich der Körperhygiene bzw. Kosmetik betrifft die Erfindung jedoch auch insbesondere gattungsgemäße Spender zur Abgabe pharmazeutischer Medien. So haben gattungsgemäße Spender auch als Nasenspray-Spender Verbreitung gefunden.

Insbesondere bei diesen Nasenspray-Spendern, jedoch auch bei anderweitigen gattungsgemäßen Spendern, wird es als nachteilig empfunden, dass der Austragkopf üblicherweise zum Austragen von Medium in seiner Gänze oder zumindest mit der Austragöffnung gemeinsam verlagert wird. Dies erschwert es, das Medium zielgerichtet auszutragen.

Den technologischen Hintergrund der Erfindung bilden Spender, wie sie in den Dokumenten WO 2006/013353 A1, WO 01/25116 A1, WO 01/44076 A2, FR 2900645 A1 und GB 1221191 A beschrieben sind.

### Aufgabe und Lösung

Aufgabe der Erfindung ist es, einen gattungsgemäßen Spender dahingehend weiterzubilden, dass bei diesem die Flüssigkeit auf technisch vorteilhafte Weise vom beweglichen Auslassstutzen zur ortsfesten Austragöffnung gefördert werden kann.

Die der Erfindung zugrundeliegende Aufgabe wird durch einen Spender nach Anspruch 1 gelöst.

Bei einem erfindungsgemäßen Spender ist somit vorgesehen, dass der Austragkopf gegeneinander verlagerbare Teilabschnitte umfasst. Ein erster Teilabschnitt ist zur ortsfesten Anbringung relativ zum Medienspeicher vorgesehen, so dass er bei bestimmungsgemäßer Verwendung gegenüber dem Medienspeicher nicht bewegt wird. Hierfür ist der erste Teilabschnitt vorzugsweise in Art einer Haube ausgebildet, die im Bereich einer umlaufenden Kante mit der Basiseinheit verbunden ist und das in Richtung des Austragkopfes weisende Ende der Basiseinheit überdeckt, wobei die Befestigung beispielsweise durch Schnapphaken oder dergleichen an der genannten Kante erfolgt, die in Vertiefungen an der Basiseinheit eingreifen.

Der erste Teilabschnitt umfasst auch die Austragöffnung, so dass auch diese ortsfest zum Medienspeicher der Basiseinheit verbleibt. Da ein erfindungsgemäßer Spender durch den Benutzer / Patienten vorzugsweise am Medienspeicher gehalten und geführt wird, kann die hierzu ortsfeste Austragöffnung demnach auf einfache Weise richtig positioniert werden.

Gegenüber dem ersten Teilabschnitt beweglich ist ein zweiter Teilabschnitt des Austragkopfes ausgebildet, welcher über eine zur manuellen Betätigung vorgesehene Betätigungshandhabe verfügt. Durch die Verlagerung dieses zweiten Teilabschnitts gegenüber dem ersten Teilabschnitt kann auch eine Verlagerung des Kopplungsstücks gegenüber dem ersten Teilabschnitt verursacht werden, was wiederum das Ausströmen von Medium aus dem Medienspeicher gestattet. Im einfachsten Falle sind die Betätigungshandhabe und das Kopplungsstück derart miteinander gekoppelt, dass sie ortsfest zueinander verbleiben, so dass das Kopplungsstück und damit der Auslassstutzen stets in gleichem Maße verlagert werden wie die Betätigungshandhabe. Es kann jedoch auch eine anderweitige Wirkkopplung realisiert sein, wie im Weiteren noch erläutert wird.

Der Auslassstutzen muss von Kopplungsstück nicht umgriffen werden. Ebenfalls als Auslassstutzen im Sinne der Erfindung ist ein verlagerbarer Ring zu verstehen, in den als weibliches Bauteil das Kopplungsstück eingeschoben wird.

Ein erfindungsgemäßer Spender kann auf vielfältige Art und Weise ausgebildet sein. So kann beispielsweise eine Pumpeinrichtung Teil der Basiseinheit sein, die durch Verlagerung des Auslassstutzens gegenüber dem Medienspeicher das Medium fördert.

Bevorzugt ist jedoch eine Gestaltung, bei der der Medienspeicher zur Lagerung von unter Überdruck stehende Medium ausgebildet ist und/oder mit unter Überdruck stehende Medium befüllt ist. Der Auslassstutzen der Basiseinheit eines solchen Spenders ist mit einem in der Basiseinheit vorgesehenen Auslassventil derart verbunden, dass eine Verlagerung des Auslassstutzens gegenüber dem Medienspeicher zu einem Öffnen des Auslassventils führt. In Reaktion auf dieses Öffnen des Auslassventils strömt das Medium durch den Auslassstutzen hindurch.

Der Auslassstutzen ist gegenüber der Basiseinheit vorzugsweise translativ in Haupterstreckungsrichtung des Spenders beweglich. Er kann im Einzelfall jedoch kippbeweglich oder anderweitig relativbeweglich sein. Gerade im Zusammenhang mit Spendern mit der genannten besonderen Ausgestaltung der Basiseinheit mit unter Druck stehendem Medium ist es bislang gängige Praxis, dass der Austragkopf in der oben beschriebenen Art und Weise so ausgestaltet ist, dass die Austragöffnung gegenüber dem Medienspeicher bewegt wird, wenn die Betätigungshandhabe manuell betätigt wird. Dies wird erfindungsgemäß vermieden.

Der Austragkopf eines erfindungsgemäßen Spenders, der die Austragöffnung aufweist, ist vorzugsweise derart ausgebildet, dass die Austragrichtung an der Austragöffnung mit der genannten Haupterstreckungsrichtung des Spenders einen Winkel größer 0° und kleiner 30° einschließt, vorzugsweise einen Winkel zwischen 5° und 15°. Weiterhin wird es als vorteilhaft angesehen, wenn die Austragöffnung exzentrisch am Austragkopf angeordnet ist, also nicht mit dem Auslassstutzen fluchtend.

Damit das Medium vom Auslassstutzen und dem hiermit verbundenen Kopplungsstück zur Auslassöffnung gelangen kann, ist der bereits genannte Auslasskanal vorgesehen. Da bei einem erfindungsgemäßen Spender das Kopplungsstück und die Austragöffnung im Zuge der Betätigung der Betätigungshandhabe gegeneinander verlagert werden, muss dieser Auslasskanal eine veränderliche Formgebung aufweisen. Als eine hierfür vorgesehene besonders einfache Gestaltung wird es angesehen, wenn der Auslasskanal zwischen dem Kopplungsstück und der Austragöffnung zumindest abschnittsweise durch einen formflexiblen Schlauchabschnitt gebildet wird. Das zur Austragöffnung hinweisende Ende dieses Schlauchabschnitts verbleibt ortsfest zum ersten Teilabschnitt, während das gegenüberliegende Ende des Schlauchabschnitts ortsfest zum Kopplungsstück verbleibt. Der Schlauchabschnitt kann als separates Bauteil ausgebildet sein. Er kann jedoch auch mit einem der angrenzenden Bauteile, beispielsweise dem Kopplungsstück, einstückig ausgebildet sein.

Im einfachsten Falle ist der Schlauchabschnitt mit einem einheitlichen Innen- und Außenquerschnitt ausgebildet. Um die Knickneigung des Schlauches zu verringern bzw. zu beeinflussen, kann es jedoch von Vorteil sein, wenn der Schlauchabschnitt über seine Länge einen uneinheitlichen Innen- oder Außenquerschnitt aufweist. Hierdurch kann verhindert werden, dass es zu einer lokalen Verengung des Innenquerschnitts des Schlauchabschnitts kommt, der den Medienaustrag behindert. Insbesondere von Vorteil ist eine Ausgestaltung, bei der der Außenquerschnitt mehrere Aufweitungen, beispielsweise in Form umlaufender Stege, aufweist, die im Falle einer Biegung des Schlauches im Kontakt miteinander gelangen und dadurch eine weitergehende Biegung verhindern. Ein ähnlicher Effekt ist durch tangential ausgerichtete Einkerbungen zu erzielen.

Durch die Aufweitungen wird eine maximale Verformbarkeit im Bereich zweier benachbarter Aufweitungen definiert. Zwar wird eine hierüber hinausgehende Verformbarkeit bei gleichzeitigem ungewünschten Einknicken des Schlauchs nicht vollständig verhindert. Ein entsprechend ausgestalteter Schlauch sorgt jedoch dafür, dass vor einer fortgesetzten Verformung in einem Teilabschnitt des Schlauchs zunächst eine Verformung in anderen Teilabschnitten des Schlauchs stattfindet, so dass ein vollständiges Verschließen des Austragkanals verhindert wird.

Erfindungsgemäß ist vorgesehen, dass der Austragkanal zwischen dem Auslassstutzen und dem Kopplungsstück einerseits und der Austragöffnung andererseits zumindest partiell durch einen teleskopierbaren Kanalbereich gebildet wird. Im Bereich des teleskopierbaren Kanalbereichs sind zwei gegeneinander verschiebliche Kanalabschnitte vorgesehen, von denen einer in den anderen eintauchend eine variable Länge des teleskopierbaren Kanalbereichs ermöglicht. Die beiden Kanalabschnitte sind dabei umlaufend in Berührkontakt miteinander, um eine wirksame Abdichtung des Kanals gegenüber einer Umgebung zu erzielen.

Vorzugsweise ist der teleskopierbare Kanalbereich in Haupterstreckungsrichtung des Spenders ausgerichtet. Im Falle einer Basiseinheit mit translativ verlagerbarem Auslassstutzen ist der teleskopierbare Kanalbereich vorzugsweise parallel zu der Verlagerungsrichtung des Auslassstutzens teleskopierbar.

Eine ebenfalls als vorteilhaft angesehene Lösung zur Erzielung der Formveränderlichkeit des Austragkanals sieht vor, dass dieser partiell durch ein Innenrohr und eine außenseitig auf dem Innenrohr verschiebliche Hülse gebildet wird, wobei sowohl das Innenrohr als auch die Hülse eine Durchbrechung aufweisen, durch die Medium in Richtung der Austragöffnung strömen kann. Dabei sind die Durchbrechungen vorzugsweise mit einer ausreichenden Größe zu versehen, durch die eine permanente Verbindung zwischen einem Kanalabschnitt, der sich an die Hülse anschließt, und dem Inneren des Innenrohrs gegeben ist.

Grundsätzlich ist bei einem erfindungsgemäßen Spender eine Gestaltung möglich, bei der der zweite Teilabschnitt, der die Betätigungshandhabe umfasst, stets ortsfest zum Kopplungsstück verbleibt. In einem solchen Falle stellt die Führung der zweiten Teileinheit an der ersten Teileinheit gleichzeitig eine Führung für das Kopplungsstück dar.

Bevorzugt wird jedoch eine Gestaltung, bei der keine, beispielsweise durch eine Einstückigkeit oder feste Verbindung gegebene, permanente Ortsfestigkeit zwischen der zweiten Teileinheit und dem Kopplungsstück vorgesehen ist. In einem solchen Falle ist es von Vorteil, wenn dennoch eine Führung für das Kopplungsstück vorgesehen ist.

Eine Gestaltung einer solchen Führung sieht vor, dass an der zum Austragkopf hinweisenden Seite der Basiseinheit ein eine zylindrische Außenwandung der Basiseinheit endseitig verschließender Deckel vorgesehen ist, wobei dieser Deckel eine Führungsfläche definiert, an der mit dem Kopplungsstück fest verbundene Führungsabschnitte gleitend beweglich sind.

Bei dem genannten Deckel kann es sich vorzugsweise um einen Crimpdeckel handeln, der endseitig den in etwa zylindrischen Medienspeicher der Basiseinheit verschließt. Der Deckel stellt die genannte Führungsfläche zur Verfügung, korrespondierend zu welcher die Formgebung von Führungsabschnitten des Kopplungsstücks geartet ist. Im montierten Zustand ist daher das Kopplungsstück durch die Führungsfläche und die mit ihm verbundenen Führungsabschnitte geführt beweglich, insbesondere translativ geführt beweglich. Bei der Führungsfläche auf Deckelseite kann es sich vorzugsweise um eine zylindrische Führungsfläche handeln, wobei es sich sowohl um eine innenzylindrische als auch um eine außenzylindrische Fläche handeln kann, an der die Führungsabschnitte anliegen. Grundsätzlich kann die Führungsfläche auch eine von der Zylinderform abweichende Formgebung aufweisen, wobei in diesem Falle Führungsabschnitte auf Seiten des Kopplungsstücks erforderlich sind, die eine korrespondierende Verformbarkeit gestatten.

Die genannte Führung des Kopplungsstücks am Deckel der Basiseinheit stellt zwar im Betrieb die gewünschte Führung des Kopplungsstücks zur Verfügung. Sie kann jedoch nur in begrenztem Maße der Positionierung des Kopplungsstücks während der Montage des Spenders dienen.

Eine diesbezüglich vorteilhafte Gestaltung sieht vor, dass der Spender eine vollständig im Austragkopf realisierte Führungseinrichtung aufweist, die dafür ausgebildet ist, eine Bewegung des Kopplungsstücks gegenüber dem ersten Teilabschnitt nur entlang eines definierten Bewegungsfalles zu ermöglichen. Eine solche vollständig im Austragkopf realisierte Führungseinrichtung führt zu einer gewünschten Positionierung des Kopplungsstücks bereits vor der Ankopplung des Austragkopfes an die Basiseinheit. Hierdurch wird die Montage erleichtert, da keine gesonderten Maßnahmen erforderlich sind, um das Kopplungsstück fluchtend mit dem Auslassstutzen zu positionieren.

Die Führungseinrichtung ist dabei vorzugsweise als Gleitführung mit mindestens zwei aufeinander angepassten Gleitführungsabschnitten ausgebildet, die gegeneinander linear verlagerbar sind. Bei einer bevorzugten Gestaltung erstrecken sich zwischen dem das Kopplungsstück umgebenden ersten Teilabschnitt des Austragkopfes und dem Kopplungsabschnitt eine Mehrzahl von Speichen, die entweder fest mit dem ersten Teilabschnitt oder fest mit dem Kopplungsstück verbunden sind und die endseitig in Nuten am Kopplungsstück bzw. am ersten Teilabschnitt geführt sind.

Zur Positionierung des Kopplungsstücks bereits vor Montage des Austragkopfes an der Basiseinheit sieht eine andere vorteilhafte Ausgestaltung vor, dass mindestens eine, vorzugsweise mindestens zwei verformbare Brücken vorgesehen sind, die einerseits mit dem Kopplungsstück verbunden sind und die andererseits ortsfest zum ersten Teilabschnitt des Austragkopfes gehalten sind. Diese Brücken weisen somit jeweils ein Ende auf, welches beispielsweise durch einstückige Ausgestaltung, ortsfest zum Kopplungsstück verbleibt. Das jeweilige gegenüberliegende andere Brückenende verbleibt ortsfest zum ersten Teilabschnitt, beispielsweise indem es mit diesem einstückig ausgebildet ist oder indem es an einem ortsfest zum ersten Teilabschnitt befestigten Halteabschnitt wie einem Haltering verbunden ist. Auch solche verformbaren Brücken, die durch ihre Verformbarkeit die Relativbewegung des Kopplungsstücks gegenüber dem ersten Teilabschnitt gestatten, können während der Montage die Positionierung des Kopplungsstücks und damit die einfache Montierbarkeit gewährleisten. Vorzugsweise sind mehrere Brücken vorgesehen, die sich radial von außen zum Kupplungsstück erstrecken.

Wie bereits erläutert, ist beim Austragkopf eines erfindungsgemäßen Spenders vorzugsweise vorgesehen, dass keine feste Verbindung zwischen dem zweiten Teilabschnitt und dem Kopplungsstück gegeben ist, so dass der Bewegungspfad des Kopplungsstücks gegenüber dem ersten Teilabschnitt nicht dem Bewegungspfad des zweiten Teilabschnitts mit Betätigungshandhabe gegenüber dem ersten Teilabschnitt entsprechen muss. Die Wirkkopplung zwischen der Verlagerung des zweiten Teilabschnitts gegenüber dem ersten Teilabschnitt einerseits und der Verlagerung des Kopplungsstücks gegenüber dem ersten Teilabschnitt andererseits erfolgt vorzugsweise zumindest auch über einen Schwenkhebel, der schwenkbar am ersten Teilabschnitt gelagert ist und der durch Verlagerung des zweiten Teilabschnitts gegenüber dem ersten Teilabschnitt verschwenkbar ist. Dieser ist mit dem Kopplungsstück derart wirkgekoppelt, dass die Schwenkbewegung des Schwenkhebels auch die Verlagerung des Kopplungsstücks bewirkt.

Die Verwendung eines solchen Schwenkhebels bietet eine Mehrzahl von Vorteilen. Sie gestattet insbesondere eine vergleichsweise flexible Anordnung des zweiten Teilabschnitts, da dieser nicht mehr zumindest in etwa mit dem Auslassstutzen und dem ersten Kopplungsstück fluchten muss. Darüber hinaus kann erreicht werden, dass es einer vergleichsweise weiten Bewegung des zweiten Teilabschnitts bedarf, um die zumeist nur geringe Bewegung des Kopplungsstücks zu bewirken, welche zum Zwecke des Medienaustrags erforderlich ist. So kann der Austragvorgang gut dosiert werden.

Der Schwenkhebel kann als separates Bauteil ausgebildet sein, welches durch Scharniermittel am ersten Teilabschnitt befestigt ist. Auch eine einstückige Gestaltung mit einem Filmscharnier ist denkbar.

Neben den genannten Vorteilen kann die Verwendung eines Schwenkhebels auch von Vorteil sein, um die gewünschte Positionierung und/oder Führung des Kopplungsstücks zu bewirken. Zu diesem Zweck kann das Kopplungsstück fest oder schwenkbar am Schwenkhebel befestigt sein. Zwar führt eine derartige Kopplung dazu, dass das Kopplungsstück entsprechend der Beweglichkeit des Schwenkhebels nicht linearbeweglich ist. Selbst bei einer Gestaltung der Basiseinheit mit linear beweglichem Auslassstutzen stellt dies jedoch kein Problem dar, da der Schwenkhebel vorzugsweise um nicht mehr als maximal 20° verschwenkt wird. Bei geeigneter Anordnung der Schwenkachse ist die Verlagerung des Kopplungsstücks quer zur Bewegungsrichtung des Auslassstutzens daher vernachlässigbar klein.

Grundsätzlich kann der zweite Teilabschnitt des Spenders ebenso wie der erste Teilabschnitt aus einem vergleichsweise starren Kunststoff gefertigt sein. In diesem Falle ist vorzugsweise eine Gleitführung zwischen den beiden Teilabschnitten vorgesehen. Eine bevorzugte Gestaltung sieht jedoch vor, dass der zweite Teilabschnitt zumindest partiell aus einem elastisch verformbaren Material besteht (E-Modul < 0,5 kN/mm²), aufgrund dessen die Betätigungshandhabe gegenüber dem ersten Teilabschnitt beweglich ist. Vorzugsweise ist der zweite Teilabschnitt als Ganzes aus diesem Material gefertigt. Weiterhin ist vorzugsweise der zweite Teilabschnitt in Randbereichen fest mit dem ersten Teilabschnitt verbunden und somit in Randbereichen gegenüber diesem unbeweglich. Die Herstellung eines solchen Austragkopfes mit einem zweiten Teilabschnitt aus elastisch verformbarem Material erfolgt vorzugsweise durch Zwei-Komponenten-Spritzguss, so dass der erste und der zweite Teilabschnitt einstückig miteinander verbunden sind.

Die Wirkkopplung zwischen dem Kopplungsstück und dem zweiten Teilabschnitt kann dergestalt sein, dass die Verlagerung bzw. Verformung des zweiten Teilabschnitts zu einer Kraftbeaufschlagung eines mit dem Kopplungsstück wirkgekoppelten oder mit diesem verbundenen Abschnitts führt, ohne dass eine permanente Verbindung zwischen dem zweiten Teilabschnitt und dem genannten dem Kopplungsstück Abschnitt erforderlich ist. Es wird jedoch als vorteilhaft angesehen, wenn ein ortsfest zum Kopplungsstück vorgesehener Befestigungsabschnitt vorgesehen ist, der kraftschlüssig, formschlüssig oder stoffschlüssig mit dem elastisch verformbaren Material des zweiten Teilabschnitts verbunden ist. Auch hierdurch kann eine Positionierung des Kopplungsstücks zum Zwecke einer erleichterten Montage erzielt werden. Insbesondere kann sich im elastisch verformbaren Material eine Aussparung befinden, in die ein Einfügeabschnitt des Befestigungsabschnitts hineinragt.

Um das Kopplungsstück im Zuge einer Betätigung der Betätigungshandhabe und einer damit einhergehenden Bewegung des zweiten Teilabschnitts gegenüber dem ersten Teilabschnitt zu verlagern, verfügt dieses vorzugsweise über mindestens eine Druckfläche. Dabei kann bei einer bevorzugten Ausgestaltung vorgesehen sein, dass die mindestens eine Druckfläche radial versetzt zum Auslassstutzen angeordnet ist. Bezogen auf die Haupterstreckungsrichtung fluchtet bei einer solchen Gestaltung die Druckfläche somit nicht mit dem Auslassstutzen. Hierdurch wird es möglich, ohne Beeinträchtigung des Verlaufs des Auslasskanals zwischen dem Kopplungsstück und der Austragöffnung die zum Zwecke eines Austragvorgangs erforderliche Kraftbeaufschlagung des Kopplungsstücks zu bewirken. Es ist jedoch auch denkbar, die Druckfläche fluchtend zum Auslassstutzen anzuordnen, wobei in diesem Fall der Austragkanal im Bereich des Kopplungsstücks und unterhalb der Druckfläche umgelenkt wird, so dass er seitlich an der mit dem Auslassstutzen fluchtenden Druckfläche vorbeigeführt wird.

Die Erfindung betrifft als Weiterbildung der beschriebenen Gestaltung, einen beschriebenen Spender, bei dem der Austragkanal zumindest abschnittsweise durch zwei durch Zwei-Komponenten-Spritzguss einstückig miteinander ausgebildete Kunststoffbauteile aus verschiedenem Kunststoff begrenzt wird, von denen der weichere Kunststoff ein E-Modul von maximal 0,3 kN/mm² aufweist. Von den beiden Kunststoffteile aus jenem Kunststoff ist eines aus dem genannten weichen Kunststoff mit einem E-Modul von maximal 0,3 kN/mm² gefertigt, während das andere vorzugsweise aus einem deutlich starreren Kunststoff gefertigt ist, insbesondere einem Kunststoff mit einem E-Modul von mindestens 1 kN/mm². Um trotz des Zwei-Komponenten-Spritzgießens zu verhindern, dass der Austragkanal verschlossen wird, wird vorzugsweise zunächst das aus dem starreren Kunststoff bestehende Bauteil hergestellt und nachfolgend im Bereich des späteren Austragkanals mit einer Beschichtung oder einer Strahlenbehandlung behandelt, um beim Anspritzen der weicheren Komponente in diesem Bereich die Herstellung eines Stoffschlusses zwischen den beiden Kunststoffbauteilen zu verhindern.

Die genannte zumindest abschnittsweise Bildung des Austragkanals durch die beiden im Zwei-Komponenten-Spritzguss miteinander verbundenen Kunststoffbauteile bietet viele Vorteile. So kann hierdurch beispielsweise auf einfache Weise ein Austragkanal geschaffen werden, der gleichzeitig eine Ventilfunktion erfüllt.

Weiterhin kann, wenn das Kunststoffbauteil aus weicherem Kunststoff zumindest abschnittsweise an der Außenseite des Spenders vorgesehen ist, dieses gleichzeitig die Betätigungshandhabe bilden. Neben der Tatsache, dass hierdurch ein erfindungsgemäßer Spender mit einer geringeren Zahl von Bauteilen hergestellt werden kann, ist diese Gestaltung auch deshalb von Vorteil, da sie es gestattet, den Medienaustrag auch haptisch im Bereich der Betätigungshandhabe erkennbar zu machen. Durch die beim Austrag stattfindende Aufweitung des Auslasskanals kann der Bediener spüren, dass der Austrag stattfindet.

### Kurzbeschreibung der Zeichnungen

Weitere Aspekte und Vorteile der Erfindung ergeben sich außer aus den Ansprüchen auch aus der nachfolgenden Beschreibung von bevorzugten Ausführungsbeispielen. Dabei zeigen:
- Fig. 1: den Grundaufbau eines erfindungsgemäßen Spenders und
- Fig. 2a bis 3b: zwei nicht von der Erfindung umfasste Varianten des Austragkopfes eines Spenders nach Fig. 1 und
- Fig. 4a bis 6b: drei erfindungsgemäße Variationen des Austragkopfes eines erfindungsgemäßen Spenders.

### Detaillierte Beschreibung der Ausführungsbeispiele

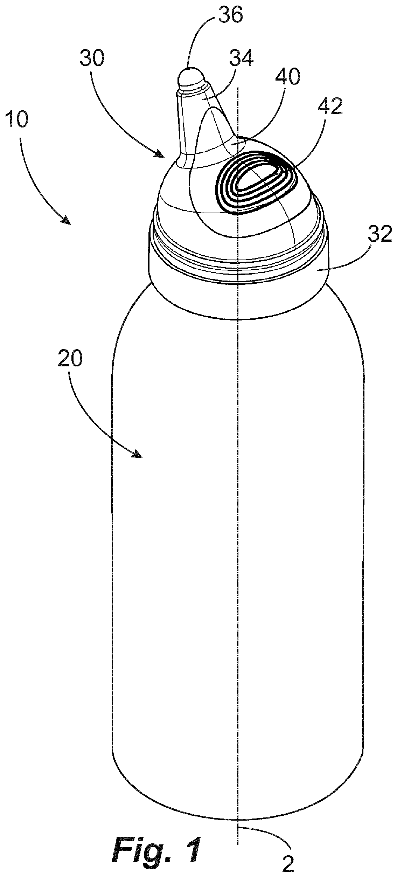

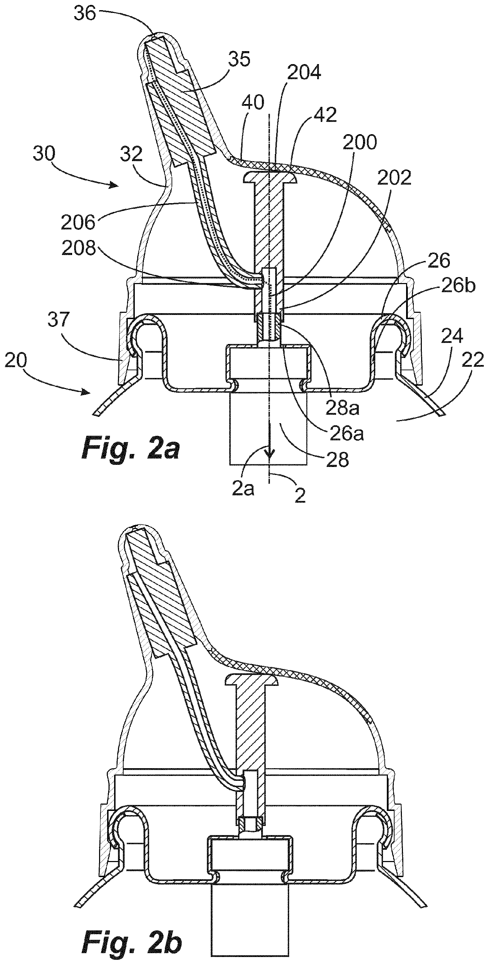

Figur 1 zeigt den Grundaufbau eines Spenders. Anhand dieser Figur sowie des ersten hinsichtlich des Austragkopfes dieses Spenders präziseren Ausführungsbeispiels der Figuren 2a und 2b wird zunächst der Grundaufbau des Spenders erläutert, der für alle hier beschriebenen erfindungsgemäßen Ausgestaltungen zumindest weitgehend identisch ist.

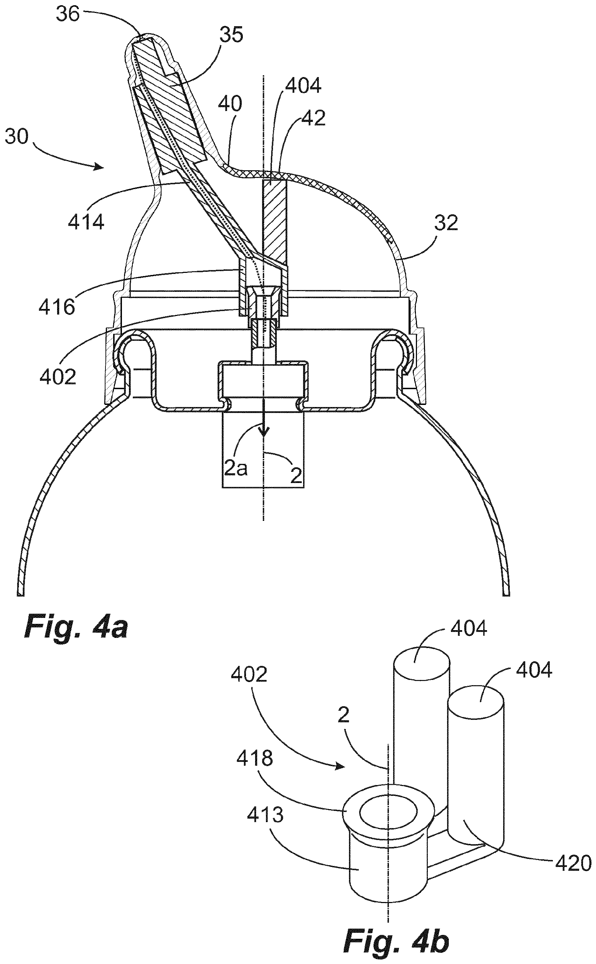

Die Gestaltungen der Fig. 2a bis 3b sind nicht von der Erfindung umfasst und nur zum Zwecke der Erläuterung der Erfndung in dieser Beschreibung beschrieben. Die Gestaltungen der Fig. 4a bis 6b bilden die erfindungsgemäßen Gestaltungen.

Der in Fig. 1 dargestelte Spender 10 verfügt über eine Basiseinheit 20 sowie einen hierauf aufgesetzten Austragkopf 30. Die Basiseinheit 20 wird zum überwiegenden Teil durch einen Medienspeicher 22 gebildet, der im Falle der Ausführungsbeispiele durch einen zu einer Haupterstreckungsachse 2 rotationssymmetrischen Druckbehälter 24 umgeben ist. Am oberen Stirnende des Druckbehälters 24 ist dieser durch einen Crimpdeckel 26 verschlossen. Der Crimpdeckel 26 wiederum weist zentrisch eine Durchbrechung 26a auf, durch die ein Auslassstutzen 28a eine Auslassventileinheit 28 hindurchragt. Der Auslassstutzen 28a ist gegen die Kraft einer nicht dargestellten Ventilfeder der Auslassventileinheit 28 in Richtung des Pfeils 2a verlagerbar, wobei durch eine derartige Verlagerung ein Öffnen der Ventileinheit 28 und damit ein Austreten der Flüssigkeit aus dem Flüssigkeitsspeicher 22 ermöglicht wird. Der Auslassstutzen 28a ist hohlzylindrisch ausgebildet. Das aus dem Medienspeicher 22 austretende Medium strömt durch den Auslassstutzen 28a hindurch.

Auf die Basiseinheit 20 ist der bereits genannte Austragkopf 30 aufgesetzt. Dieser Austragkopf 30 verfügt über einen zum Druckkörper 24 ortsfesten ersten Teilabschnitt 32, welcher den überwiegenden Teil des Austragkopfes 30 bildet. Er umfasst neben der Befestigungseinrichtung 37 zum Zwecke der Befestigung des ersten Teilabschnitts am Druckkörper 24 auch die Austragöffnung 36, durch die das Medium, insbesondere eine Flüssigkeit in Form von Sprühstrahls, in eine umgebende Atmosphäre ausgetragen werden kann. Die Austragöffnung ist im Falle des in Ausführungsbeispielen vorgesehenen Spenders 10 am distalen Ende eines konischen Nasenapplikators 34 vorgesehen, welcher gegenüber der Haupterstreckungsachse 2 des Spenders 10 in einem Winkel von etwa 10° bis 15° angewinkelt ausgerichtet ist und innerhalb dessen ein zum Applikator 34 ortsfester Einsatz 35 vorgesehen ist.

Der Austragkopf 30 verfügt darüber hinaus über einen zweiten Teilabschnitt 40, welcher gegenüber dem ersten Teilabschnitt 32 relativbeweglich ist. Im Falle der vorliegenden Ausführungsbeispiele wird dieser zweite Teilabschnitt zumindest partiell durch ein Bauteil aus einem vergleichsweise weichen und daher verformbaren thermoplastischen Kunststoff gebildet, welches in Randbereichen am ersten Teilabschnitt 32 befestigt ist, beispielsweise durch Zwei-Komponenten-Spritzguss mit diesem verbunden ist. Der zweite Teilabschnitt 40, der gegenüber dem ersten Teilabschnitt 32 und dem Druckbehälter 24 beweglich ist, ist auch Träger einer Betätigungshandhabe 42, welche zum Zwecke des Austrags von Medium hinabgedrückt werden kann.

Die dargestellte Ausgestaltung mit einer Einstückigkeit zwischen dem ersten Teilabschnitt und dem zweiten Teilabschnitt bzw. der Betätigungshandhabe kann auch ersetzte werden durch eine Gestaltung mit vollständig separater Gestaltung des zweiten Teilabschnitts, bei der dieser gegenüber dem ersten Teilabschnitt geführt beweglich ist.

Die Handhabung des Spenders erfolgt derart, dass die Austragöffnung 36 in der gewünschten Austragposition positioniert wird. Anschließend wird die Betätigungshandhabe 42 gegenüber dem Druckkörper 24, der gleichzeitig zum Halten des Spenders 10 dient, hinabgedrückt. Hierdurch wird ein Flüssigkeitsaustrag bewirkt, wobei dies nicht mit einer Verlagerung der Austragöffnung 36 gegenüber dem Druckbehälter 24 einhergeht. Diese Unbeweglichkeit der Austragöffnung 36 gegenüber dem Druckbehälter 24 ist bei vielen Anwendungszwecken von Vorteil, beispielsweise bei einer Verwendung eines dargestellten Spenders 10 als Nasenspray-Spender, bei dem durch die Unbeweglichkeit verhindert wird, dass der Applikatior 34 im Zuge der Betätigung innerhalb eines Nasenlochs des Benutzers eine Verlagerung erfährt.

Nachfolgend werden verschiedene Ausgestaltungen des Austragkopfes erläutert. Bei diesen Ausführungsformen sind jeweils verschiedene einstückige Bauteile vorgesehen. Soweit bei den Ausführungsformen verschiedene Teilabschnitte eines Bauteils durch Einstückigkeit ortsfest zueinander ausgebildet sind, ist dies jedoch lediglich beispielhaft zu verstehen. Die entsprechenden Bauteile können auch mehrteilig durch miteinander verbunden Teilbauteile gebildet sein.

Bei der Ausgestaltung gemäß den Figuren 2a und 2b ist ein Kopplungsstück 202 vorgesehen, welches auf den Auslassstutzen 28a aufgesetzt ist. Dieses Kopplungsstück weist an seinem dem Auslassstutzen 28a gegenüberliegenden Ende eine Druckbeaufschlagungsfläche 204 auf, welche unterhalb des zweiten Teilabschnitts 40 angeordnet ist und durch ein Hinabdrücken des zweiten Teilabschnitts 40 bzw. der daran vorgesehenen Betätigungshandhabe 42 ebenfalls in Richtung des Pfeils 2a nach unten verlagert werden kann. Das Kopplungsstück 202 ist dabei in einem unteren Bereich hohl ausgebildet und weist eine sich radial nach außen erstreckende Kopplungsbohrung 208 auf, in die ein formveränderlicher Schlauchabschnitt 206 eingeschoben ist. Bei einer alternativen Ausgestaltung kann der Schlauchabschnitt 206 auch einstückig mit dem Kopplungsstück 202 ausgebildet sein. Das gegenüberliegende Ende des Schlauchabschnitts 206 endet an dem Füllstück 35, das innerhalb des Applikators 34 ortsfest angeordnet ist. Das Füllstück 35 ist mit einer Bohrung versehen, die Teil des Austragkanals 200 ist, durch den hindurch das Medium vom Auslassstutzen 28a bis zur Austragöffnung 36 gelangen kann. Wie Figur 2b verdeutlicht, bedarf es keiner unmittelbaren und dauerhaften Kopplung zwischen dem zweiten Teilabschnitt 40 und dem Kopplungsstück 202, um die Verlagerung des Kopplungsstücks 202 mittels der Betätigungshandhabe 42 bewirken zu können.

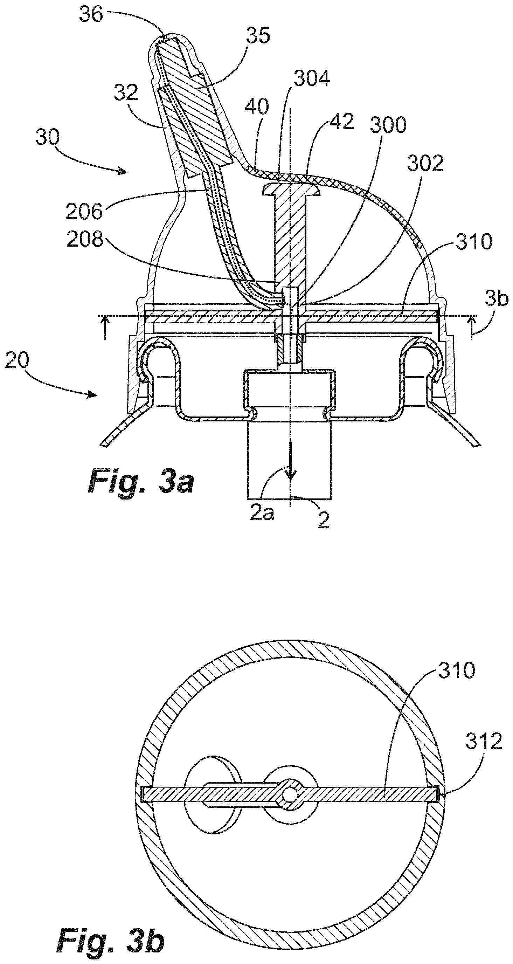

Die Ausgestaltung gemäß der Figuren 3a und 3b stellt eine Variante zum Ausgestalten der Figuren 2a und 2b dar. Diese Variante wird in Figur 3a in einer geschnittenen Seitenansicht und in Figur 3b in einer geschnittenen Ansicht von unten verdeutlicht. Bei dieser Gestaltung sind zwei einstückig am Kopplungsstück 302 vorgesehene Speichen 310 vorgesehen, die sich radial nach außen erstrecken. Die äußeren Enden dieser Speichen 310 ragen in nach innen weisende Nuten 312, die an der Innenseite des ersten Teilabschnitts oder hierzu ortsfest vorgesehen sind und die sich parallel zur Haupterstreckungsachse 2 erstrecken.

Durch die Führung, die durch die Speichen 310 und die Nuten 312 gebildet wird, ist im Betrieb eine höhere Funktionssicherheit gewährleistet, da die Gefahr verringert wird, dass das Kopplungsstück 302 sich vom Auslassstutzen 28a löst. Die Führung sorgt jedoch bereits zuvor, während der Montage des Spenders 10, für Vorteile, da sie es gestattet, ohne eine gesonderte Ausrichtung den Austragkopf nach dessen Montage auf die Basiseinheit 20 aufzusetzen. Durch die Speichen ist eine ausreichend genaue Positionierung des Kopplungsstücks 302 erzielbar.

Die Ausgestaltung gemäß den Figuren 4a und 4b verzichtet auf einen verformbaren Schlauch, wie ihn die vorangegangenen Figuren zeigen. Stattdessen ist der Austragkanal 400, der den Auslassstutzen 28a mit der Austragöffnung 36 verbindet, in einem Teilbereich teleskopierbar ausgebildet. In diesem Teilbereich befindet sich zum einen ein Kanalabschnitt 413 des Kopplungsstückes 402, welches in ortsfester Art und Weise auf dem Auslassstutzen 28a aufgesetzt ist. Zum anderen befindet sich hier ein in Haupterstreckungsrichtung 2 ausgerichteter Kanalabschnitt 416, der ortsfest, vorliegend einstückig mittels eines Kanalabschnitts 414, mit dem Einsatz 35 verbunden ist. Der kopplungsstückseitige Kanalabschnitt 413 erstreckt sich in den zum ersten Teilabschnitt 32 ortsfesten hohlzylindrischen Abschnitt 416, wobei durch eine sich nach außen aufweitende Dichtlippe 418 gewährleistet ist, dass in diesem Kontaktbereich keine Flüssigkeit austreten kann. Das Kopplungsstück 402 ist in Figur 4b nochmals dargestellt. Aus dieser Figur ist auch ersichtlich, dass als einstückiger Teil des Kopplungsstückes 402 nach oben weisende und exzentrisch zur Hauptachse 2 angeordnete Fortsätze 420 vorgesehen sind. Diese Fortsätze 420 erstrecken sich bis unmittelbar unter die Betätigungshandhabe 42. Es ist daher möglich, durch Niederdrücke der Betätigungshandhabe 42 das im Teleskopbereich geführte Kopplungsstück 402 hinabzudrücken, um dadurch auch den Auslassstutzen 28a hinabzudrücken. Hierdurch wird der Austragvorgang bewirkt. Die Flüssigkeit strömt durch den Teleskopabschnitt zur Austragöffnung 36.

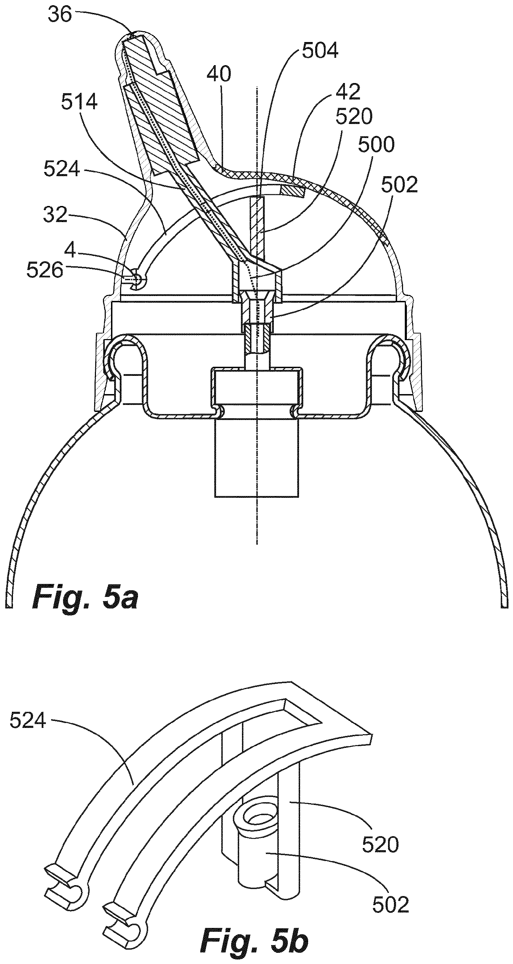

Die Ausgestaltung der Figuren 5a und 5b ähnelt der Ausgestaltung der Figuren 4a und 4b weitgehend. Allerdings ist hier vorgesehen, dass ein Niederdrücken der Betätigungshandhabe 42 nicht unmittelbar auf das Kopplungsstück 502 wirkt. Stattdessen ist ein Zwischenhebel 524 vorgesehen, der um eine zum ersten Teilabschnitt 32 ortsfeste Schwenkachse 4 schwenkbeweglich gelagert ist. Dieser Schwenkhebel 524 drückt, sobald er mittels der Betätigungshandhabe 42 nach unten verlagert wurde, auf die Stirnflächen 504 der Fortsätze 520 und bewirkt so den Austragvorgang.

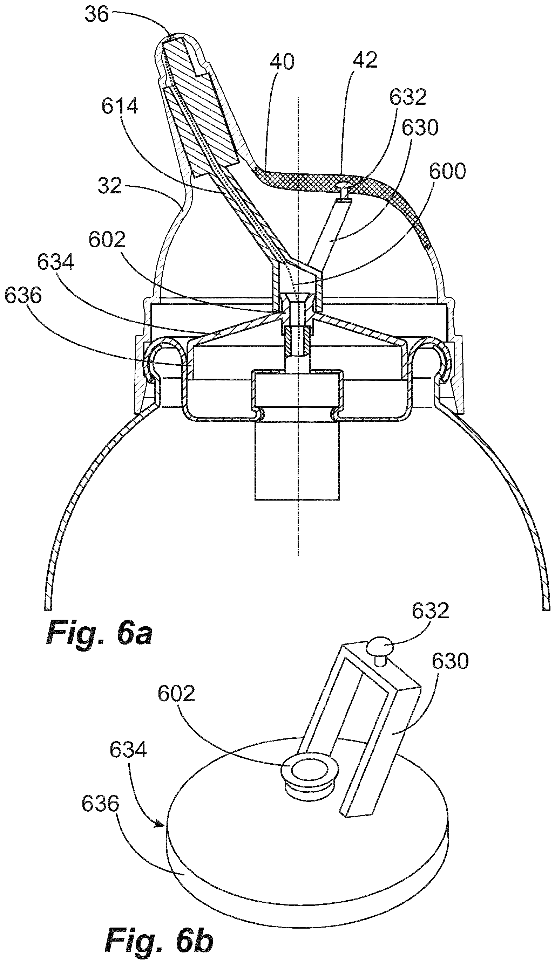

Bei der Ausgestaltung gemäß der Figuren 6a und 6b wirkt, ähnlich wie bei der Ausgestaltung der Figuren 4a und 4b, die Betätigungshandhabe ohne Zwischenschaltung eines Schwenkhebels auf das Kopplungsstück 602. Hierfür ist am Kopplungsstück 602 ein sich zur Betätigungshandhabe 42 erstreckender Betätigungsfortsatz 630 vorgesehen, an dessen distalen Ende eine pilzförmige Aufweitung 632 angebracht ist. Diese ist in einer Aussparung an der Innenseite der Betätigungshandhabe 42 eingesetzt. Somit ist bereits vor Anbringung des Austragkopfes 30 auf der Basiseinheit 20 eine zumindest ungefähre Positionierung des Kopplungsstücks 650 gegeben, die eine erleichterte Montage gestattet. Als zusätzliche Besonderheit ist bei der Ausgestaltung der Figuren 6a und 6b vorgesehen, dass das Kopplungsstück 650 einen Führungskragen 634 aufweist, dessen Außenfläche 636 im montierten Zustand mit einer zylindrischen Führungsfläche 26b des Crimpdeckels 26 zusammenwirkt. Durch diese Maßnahmen sowie durch die Teleskopführung des Kopplungsstücks 602, die der der Figuren 4a und 4b weitgehend entspricht, wird eine zuverlässige Führung des Kopplungsstücks 602 im Betrieb erzielt.

## Patentansprüche

1. Spender (10) zum Austrag von Medien, insbesondere von Flüssigkeiten, mit
- einer Basiseinheit (20), umfassend:
- einen Medienspeicher (22), der zur Lagerung von Medium ausgebildet ist, und
- eine Auslassstutzen (28a), der gegenüber dem Medienspeicher (22) bewegbar ist, um hierdurch einen Austrag von Medium in Richtung eines Austragkopfes (30) durch den Auslassstutzen (28a) hindurch zu verursachen, und
- einem Austragkopf (30), umfassend:
- eine Austragöffnung (36), durch die hindurch das Medium in eine umgebende Atmosphäre entweichen kann,
- einen Austragkanal (400; 500; 600), an dessen erstem Ende ein Kopplungsstück (402; 502; 602), zur Ankopplung an den Auslassstutzen (28a) vorgesehen ist und dessen zweites Ende mit der Austragöffnung (36) verbunden ist,
wobei der Austragkopf (30)
- einen ersten Teilabschnitt (32) aufweist, der eine Befestigungseinrichtung (37) aufweist, mittels derer er ortsfest zum Medienspeicher (20) an der Basiseinheit (10) befestigt ist und der die Austragöffnung (36) umfasst, und
- einen zweiten Teilabschnitt (40) aufweist, der beweglich gegenüber dem ersten Teilabschnitt (32) ausgebildet ist und eine Betätigungshandhabe (42) aufweist, die mit dem Kopplungsstück (402; 502; 602) derart wirkgekoppelt ist, dass eine Verlagerung der Betätigungshandhabe (42) gegenüber dem ersten Teilabschnitt (32) zu einer Verlagerung des Kopplungsstücks gegenüber dem ersten Teilabschnitt (40) führt,
**dadurch gekennzeichnet, dass**
der Austragkanal (400; 500; 600) zwischen dem Auslassstutzen (28a) und der Austragöffnung (36) zumindest partiell durch einen teleskopierbaren Kanalbereich (416, 418) gebildet wird.

2. Spender nach Anspruch 1,
**dadurch gekennzeichnet, dass**
- der Medienspeicher (22) zur Lagerung von unter Überdruck stehendem Medium ausgebildet ist und/oder mit unter Überdruck stehendem Medium befüllt ist und
- der Auslassstutzen (28a) mit einem in der Basiseinheit (20) vorgesehenen Auslassventil (28) derart verbunden ist, das eine Verlagerung des Auslassstutzens (28a) gegenüber dem Medienspeicher (22) zu einem Öffnen des Auslassventils (28) führt.

3. Spender nach Anspruch 1 oder 2,
**dadurch gekennzeichnet, dass**
der Austragkanal zwischen dem Kopplungsstück und der Austragöffnung zumindest abschnittsweise durch einen formflexiblen Schlauchabschnitt gebildet wird.

4. Spender nach Anspruch 3,
**dadurch gekennzeichnet, dass**
der Schlauchabschnitt über seine Länge einen uneinheitlichen Innen- oder Außenquerschnitt aufweist, um die Knickneigung zu beeinflussen oder zu verringern.

5. Spender nach einem der vorstehenden Ansprüche,
**dadurch gekennzeichnet, dass**
an der zum Austragkopf (30) hin weisenden Seite der Basiseinheit (20) ein eine zylindrische Außenwandung der Basiseinheit endseitig verschließender Deckel (26) vorgesehen ist, wobei dieser Deckel eine Führungsfläche (26b) definiert, an der mindestens ein mit dem Kopplungsstück verbundener Führungsabschnitt (636) gleitend beweglich sind.

6. Spender nach einem der Ansprüche 1 bis 4,
**dadurch gekennzeichnet, dass**
eine vollständig im Austragkopf realisierte Führungseinrichtung vorgesehen, die dafür ausgebildet ist, eine Bewegung des Kopplungsstücks gegenüber dem ersten Teilabschnitt nur entlang eines definierten Bewegungspfades zu ermöglichen.

7. Spender nach Anspruch 6,
**dadurch gekennzeichnet, dass**
die Führungseinrichtung als Gleitführung mit zwei aufeinander angepassten Gleitführungsabschnitten ausgebildet ist, die gegeneinander linear verlagerbar sind.

8. Spender nach einem der vorstehenden Ansprüchen,
**dadurch gekennzeichnet, dass**
mindestens eine verformbare Brücke vorgesehen ist, die einerseits mit dem Kopplungsstück verbunden ist und die andererseits ortsfest mit dem ersten Teilabschnitt des Austragkopfes verbunden ist.

9. Spender nach einem der vorstehenden Ansprüchen,
**dadurch gekennzeichnet, dass**
das ein Schwenkhebel (524) vorgesehen ist, der schwenkbar am ersten Teilabschnitt (32) gelagert ist und der durch Verlagerung des zweiten Teilabschnitts (40) gegenüber dem ersten Teilabschnitt (32) verschwenkbar ist, wobei der Schwenkhebel (524) derart mit dem Kopplungsstück (502) wirkgekoppelt ist, dass das Kopplungsstück (502) durch eine Schwenkbewegung des Schwenkhebels (524) gegenüber dem ersten Teilabschnitt (32) verlagerbar ist, wobei vorzugsweise das Kopplungsstück fest oder schwenkbar am Schwenkhebel befestigt ist.

10. Spender nach einem der vorstehenden Ansprüche,
**dadurch gekennzeichnet, dass**
der zweite Teilabschnitt (40) zumindest partiell aus einem elastisch verformbaren Material besteht, aufgrund dessen die Betätigungshandhabe (42) gegenüber dem ersten Teilabschnitt (32) beweglich ist.

11. Spender nach Anspruch 10,
**dadurch gekennzeichnet, dass**
ein ortsfest zum Kopplungsstück (602) vorgesehener Befestigungsabschnitt (632) vorgesehen ist, der kraftschlüssig, formschlüssig oder stoffschlüssig mit dem elastisch verformbaren Material des zweiten Teilabschnitts (40) verbunden ist.

12. Spender nach einem der vorstehenden Ansprüche,
**dadurch gekennzeichnet, dass**
das Kopplungsstück (402; 502) über mindestens eine Druckfläche (404; 504) verfügt, welche mittelbar oder unmittelbar durch die Betätigungshandhabe (42) kraftbeaufschlagt werden kann, um die Verlagerung des Auslassstutzens (28a) gegenüber dem Medienspeicher (22) zu bewirken,
wobei vorzugsweise
- die mindestens eine Druckfläche (404; 504) radial versetzt zum Auslassstutzen (28a) angeordnet ist, oder
- die mindestens eine Druckfläche fluchtend zum Auslassstutzen (28a) angeordnet ist, wobei der Austragkanal im Bereich des Kopplungsstücks und unterhalb dieser Druckfläche umgelenkt wird.

13. Spender nach einem der Ansprüche 1 bis 12 ausgebildet ist, **dadurch gekennzeichnet, dass** der Austragkanal zumindest abschnittsweise zwei durch Zwei-komponenten-Spritzguss einstückig miteinander ausgebildeten Kunststoffbauteilen aus verschiedenem Kunststoff begrenzt wird, von denen der weichere Kunststoff ein E-Modul von maximal 0,3 kN/mm² aufweist.

14. Spender nach Anspruch 13,
**dadurch gekennzeichnet, dass**
das Kunststoffbauteil aus dem weicheren Kunststoff zumindest abschnittsweise an einer Außenseite des Spenders vorgesehen ist, wobei insbesondere vorzugsweise das Kunststoffbauteil aus dem weicheren Kunststoff auch eine Betätigungshandhabe des Spenders bildet.

## Claims

1. Dispenser (10) for the delivery of media, in particular liquids, said dispenser having
- a base unit (20) which includes:
- a media storage unit (22) which is realized for storing a medium and
- an outlet connecting piece (28a) which is movable in relation to the media storage unit (22) in order, as a result, to cause a delivery of medium in the direction of a delivery head (30) through the outlet connecting piece (28a), and
- a delivery head (30) which includes:
- a delivery opening (36) through which the medium is able to escape into a surrounding atmosphere,
- a delivery channel (400; 500; 600), at the first end of which is provided a coupling piece (402; 502; 602) for coupling to the outlet connecting piece (28a) and the second end of which is connected to the delivery opening (36),
wherein the delivery head (30)
- comprises a first part portion (32) which comprises a fastening device (37), by means of which it is fastened fixed in position with respect to the media storage unit (20) on the base unit (10) and which includes the delivery opening (36) and
- comprises a second part portion (40) which is realized so as to be movable in relation to the first part portion (32) and comprises an actuating handle (42) which is operatively coupled with the coupling piece (402; 502; 602) in such a manner that a displacement of the actuating handle (42) in relation to the first part portion (32) leads to a displacement of the coupling piece in relation to the first part portion (40),
**characterized in that**
- the delivery channel (400; 500; 600) between the outlet connecting piece (28a) and the delivery opening (36) is formed at least in part by a telescopic channel region (416, 418).

2. Dispenser according to Claim 1,
**characterized in that**
- the media storage unit (22) is realized for storing over-pressurized medium and/or is filled with over-pressurized medium and
- the outlet connecting piece (28a) is connected to an outlet valve (28) which is provided in the base unit (20) in such a manner that a displacement of the outlet connecting piece (28a) in relation to the media storage unit (22) leads to opening of the outlet valve (28).

3. Dispenser according to Claim 1 or 2,
**characterized in that**
the outlet channel between the coupling piece and the delivery opening is formed at least in portions by a form-flexible hose portion.

4. Dispenser according to Claim 3,
**characterized in that**
the hose portion comprises a non-uniform inside or outside cross section over its length in order to influence or reduce the buckling tendency.

5. Dispenser according to one of the preceding claims,
**characterized in that**
a cover (26), which closes a cylindrical outside wall of the base unit (20), is provided on the side of the base unit (20) which points to the delivery head (30), wherein said cover defines a guiding face (26b) on which at least one guiding portion (636) which is connected to the coupling piece is slidingly movable.

6. Dispenser according to one of Claims 1 to 4,
**characterized in that**
there is provided a guiding device which is realized completely in the delivery head and is realized for the purpose of enabling a movement of the coupling piece in relation to the first part portion only along a defined movement path.

7. Dispenser according to Claim 6,
**characterized in that**
the guiding device is realized as a slip-in guide with two slip-in guide portions which are adapted to one another and are displaceable in relation to one another in a linear manner.

8. Dispenser according to one of the preceding claims,
**characterized in that**
there is provided at least one deformable bridge which is connected at the one end to the coupling piece and is connected in a fixed manner at the other end to the first part portion of the delivery head.

9. Dispenser according to one of the preceding claims,
**characterized in that**
there is provided a pivot lever (524) which is mounted so as to be pivotable on the first part portion (32) and which can be pivoted in relation to the first part portion (32) as a result of displacement of the second part portion (40), wherein the pivot lever (524) is operatively coupled in such a manner with the coupling piece (502) that the coupling piece (502) is displaceable in relation to the first part portion (32) as a result of a pivoting movement of the pivot lever (524), wherein preferably the coupling piece is fastened so as to be fixed or pivotable on the pivot lever.

10. Dispenser according to one of the preceding claims,
**characterized in that**
the second part portion (40) consists at least in part of an elastically deformable material, on account of which the actuating handle (42) is movable in relation to the first part portion (32).

11. Dispenser according to Claim 10,
**characterized in that**
there is provided a fastening portion (632) which is provided so as to be fixed in position in relation to the coupling piece (602) and is connected in a non-positive locking, positive locking or positively bonded manner to the elastically deformable material of the second part portion (40).

12. Dispenser according to one of the preceding claims,
**characterized in that**
the coupling piece (402; 502) has at least one pressing face (404; 504) which can be acted upon with force indirectly or directly by the actuating handle (42) in order to bring about the displacement of the outlet connecting piece (28a) in relation to the media storage unit (22), wherein preferably
- the at least one pressing face (404; 504) is arranged radially offset to the outlet connecting piece (28a), or
- the at least one pressing face is arranged in alignment with the outlet connecting piece (28a), wherein the delivery channel is deflected in the region of the coupling piece and below said pressing face.

13. Dispenser according to one of Claims 1 to 12,
**characterized in that**
the delivery channel is defined at least in portions by two plastics material components, which are realized integrally together as a result of two-component injection molding from a different plastics material, of which the softer plastics material comprises a maximum modulus of elasticity of 0.3 kN/mm².

14. Dispenser according to Claim 13,
**characterized in that**
the plastics material component of the softer plastics material is provided at least in portions on an outside surface of the dispenser, wherein in particular the plastics material component of the softer plastics material also preferably forms an actuating handle of the dispenser.

## Revendications

1. Distributeur (10) pour la délivrance de fluides, en particulier de liquides, avec
- une unité de base (20), comprenant:
- un réservoir de fluide (22), qui est configuré pour stocker des fluides, et
- un embout de sortie (28a), qui est mobile par rapport au réservoir de fluide (22), afin de provoquer ainsi une délivrance de fluide en direction d'une tête de délivrance (30) à travers l'embout de sortie (28a),
- une tête de délivrance (30), comprenant:
- une ouverture de délivrance (36), à travers laquelle le fluide peut s'échapper dans une atmosphère environnante,
- un canal de délivrance (400; 500; 600), à la première extrémité duquel il est prévu une pièce de couplage (402; 502; 602) pour le couplage à l'embout de sortie (28a), et dont la deuxième extrémité est raccordée à l'ouverture de délivrance (36),
dans lequel la tête de délivrance (30)
- présente une première section partielle (32), qui présente un dispositif de fixation (37), au moyen duquel elle est fixée sur l'unité de base (10) de façon immobile par rapport au réservoir de fluide (20) et qui comprend l'ouverture de délivrance (36), et
- présente une deuxième section partielle (40), qui est mobile par rapport à la première section partielle (32) et qui présente une manette d'actionnement (42), qui est couplée activement à la pièce de couplage (402; 502; 602), de telle manière qu'un déplacement de la manette d'actionnement (42) par rapport à la première section partielle (32) conduise à un déplacement de la pièce de couplage par rapport à la première section partielle (40),
**caractérisé en ce que** le canal de délivrance (400; 500; 600) entre l'embout de sortie (28a) et l'ouverture de délivrance (36) est formé au moins en partie par une région de canal télescopique (416, 418).

2. Distributeur selon la revendication 1, **caractérisé en ce que**
- le réservoir de fluide (22) est configuré pour le stockage de fluide se trouvant en surpression et/ou est rempli avec un fluide se trouvant en surpression et
- l'embout de sortie (28a) est raccordé à une soupape de sortie (28) prévue dans l'unité de base (20), de telle manière qu'un déplacement de l'embout de sortie (28a) par rapport au réservoir de fluide (22) conduise à une ouverture de la soupape de sortie (28).

3. Distributeur selon la revendication 1 ou 2, **caractérisé en ce que** le canal de délivrance entre la pièce de couplage et l'ouverture de délivrance est formé au moins localement par une section de tuyau de forme flexible.

4. Distributeur selon la revendication 3, **caractérisé en ce que** la section de tuyau présente sur sa longueur une section transversale intérieure ou extérieure inégale, afin d'influencer ou de réduire la tendance au pliage.

5. Distributeur selon l'une quelconque des revendications précédentes, **caractérisé en ce qu'**il est prévu sur le côté de l'unité de base (20) tourné vers la tête de délivrance (30) un couvercle (26) fermant à son extrémité une paroi extérieure cylindrique de l'unité de base, dans lequel ce couvercle définit une face de guidage (26b), sur laquelle au moins une section de guidage (636) reliée à la pièce de couplage est mobile de façon glissante.

6. Distributeur selon l'une quelconque des revendications 1 à 4, **caractérisé en ce qu'**il est prévu un dispositif de guidage réalisé entièrement dans la tête de délivrance, qui est configuré pour permettre un déplacement de la pièce de couplage par rapport à la première section partielle uniquement le long d'un chemin de déplacement défini.

7. Distributeur selon la revendication 6, **caractérisé en ce que** le dispositif de guidage est réalisé sous forme de guidage glissant avec deux parties de guidage glissant adaptées l'une à l'autre, qui sont déplaçables linéairement l'une par rapport à l'autre.

8. Distributeur selon l'une quelconque des revendications précédentes, **caractérisé en ce qu'**il est prévu au moins un pont déformable, qui est d'une part relié à la pièce de couplage et qui est d'autre part relié de façon fixe à la première section partielle de la tête de délivrance.

9. Distributeur selon l'une quelconque des revendications précédentes, **caractérisé en ce qu'**il est prévu un levier pivotant (524), qui est monté de façon pivotante sur la première section partielle (32) et qui peut pivoter par rapport à la première section partielle (32) par un déplacement de la deuxième section partielle (40), dans lequel le levier pivotant (524) est couplé activement à la pièce de couplage (502), de telle manière que la pièce de couplage (502) puisse se déplacer par un mouvement de pivotement du levier pivotant (524) par rapport à la première section partielle (32), dans lequel la pièce de couplage est de préférence fixée de façon fixe ou pivotante au levier pivotant.

10. Distributeur selon l'une quelconque des revendications précédentes, **caractérisé en ce que** la deuxième section partielle (40) se compose au moins en partie d'un matériau élastiquement déformable, en raison duquel la manette d'actionnement (42) est mobile par rapport à la première section partielle (32).

11. Distributeur selon la revendication 10, **caractérisé en ce qu'**il est prévu une partie de fixation (632) prévue de façon fixe par rapport à la pièce de couplage (602), qui est assemblée par adhérence, par emboîtement ou par une liaison matérielle au matériau élastiquement déformable de la deuxième section partielle (40).

12. Distributeur selon l'une quelconque des revendications précédentes, **caractérisé en ce que** la pièce de couplage (402; 502) comporte au moins une face de pression (404; 504), sur laquelle la manette d'actionnement (42) peut exercer indirectement ou directement une force, afin de provoquer un déplacement de l'embout de sortie (28a) par rapport au réservoir de fluide (22), dans lequel de préférence
- ladite au moins une face de pression (404; 504) est disposée avec un décalage radial par rapport à l'embout de sortie (28a), ou
- ladite au moins une face de pression est disposée dans l'alignement de l'embout de sortie (28a), dans lequel le canal de délivrance est dévié dans la région de la pièce de couplage et en dessous de cette face de pression.

13. Distributeur est configuré selon l'une quelconque des revendications 1 à 12, **caractérisé en ce que** le canal de délivrance est limité au moins localement par deux éléments en matière plastique constitués d'une matière plastique différente formés en une seule pièce l'un avec l'autre par un procédé de moulage par injection à deux composants, parmi lesquels la matière plastique plus souple présente un module E de 0,3 kN/mm² au maximum.

14. Distributeur selon la revendication 13, **caractérisé en ce que** l'élément en matière plastique constitué de la matière plastique plus souple est prévu au moins localement sur un côté extérieur du distributeur, dans lequel en particulier l'élément en matière plastique constitué de la matière plastique plus souple forme de préférence aussi une manette d'actionnement du distributeur.
